# EUROPÄISCHE PATENTSCHRIFT

(11) **EP 1 247 543 B1**
(45) Veröffentlichungstag und Bekanntmachung des Hinweises auf die Patenterteilung: **27.07.2005**
(21) Anmeldenummer: 02090127.8
(22) Anmeldetag: 28.03.2002
(51) Int. Cl.: A61N 1/05

(54) **Elektrodenleitung**
Electrode lead
Fil d'électrode

(30) Priorität: 05.04.2001 DE 10118797
(43) Veröffentlichungstag der Anmeldung: 09.10.2002
(73) Patentinhaber: BIOTRONIK Mess- und Therapiegeräte GmbH & Co Ingenieurbüro Berlin, 12359 Berlin (DE)
(72) Erfinder: Reinke, Heinrich, 91083 Baiersdorf-Hagenau (DE); Schaldach, Max, verstorben (DE)
(74) Vertreter: Eisenführ, Speiser & Partner

(56) Entgegenhaltungen:
- FR-A- 2 732 225
- US-A- 5 415 633
- US-A- 5 835 453
- US-A- 6 083 170
- US-A- 6 129 672

## Beschreibung

Die vorliegende Erfindung betrifft eine Elektrodenleitung insbesondere für eine implantierbare Elektrostimulationsvorrichtung, die zum Einführen in Körpergewebe wie Herzkammern oder Blutgefäße ausgebildet ist und Elektroden zum Stimulieren von Körpergewebe, eine Längsachse und ein distales Ende aufweist.

Elektrodenleitungen werden vorzugsweise dazu benutzt, umliegendes Körpergewebe elektrisch anzuregen oder elektrische Signale aufzunehmen. Sie werden insbesondere auch im Bereich der Koronargefäße oder Venen eingesetzt, um die Herzkontraktion zu steuern. Dazu ist es erforderlich, dass die Elektrode in das ausgewählte Gefäß eingeführt werden kann. Dies soll minimalinvasiv erfolgen, um den Patienten größere Belastungen zu ersparen. Soll die Elektrode in Koronarsinus am linken Atrium oder in einer der sich anschließenden Venen am linken Ventrikel untergebracht werden, so gestaltet sich der Zugang folgendermaßen: die Elektrode wird zunächst durch einen verhältnismäßig großvolumigen transvenösen bzw. venösen Zugang zum rechten Atrium geführt. Hierauf muss sie einen nahezu rechten Winkel beschreiben, um in den Koronarsinus zu gelangen. Soll die Elektrode tiefer in den Koronarsinus hineingeschoben werden und weitere Abbiegungen vollziehen, so ist ein Führungsdraht vorgesehen, der für die Schiebbarkeit der Elektrode sorgt. Ein weiteres Erfordemis besteht in der Lenkbarkeit der Elektrodenleitung, um die Elektrodenleitung durch gekrümmte oder verzweigte Hohlräume zu führen.

Aus US-A-5,425,633 ist ein Katheter mit einer steuerbaren Spitze bekannt, der über eine oder mehrere Lagen piezoelektrischen keramischen Materials verfügt und dessen Spitze durch Anlegen einer elektrischen Spannung an die piezokeramischen Steuerelemente gesteuert werden kann. Als alternatives elektrostriktives Material wird Polyvinylidendifluorid vorgeschlagen.

Das US-Patent 6,083,170 offenbart eine flexible, längliche Sonde, welche einen Ausrichtungsmechanismus aufweist, der das distale Ende der Sonde in Abhängigkeit von Steuersignalen verbiegt. Als steuerbare Beugungselemente werden Piezokristalle oder Formgedächtnismetalle vorgeschlagen. Elektrodenleitungen zur Stimulation von Gewebe insbesondere des Myokards sind jedoch dazu vorgesehen, dauerhaft implantiert zu werden. Die mit Piezokristallen oder Formgedächtnismetallen betriebenen Steuermechanismen sind jedoch üblicherweise nicht ausreichend langzeitstabil. Die dauerhaft implantierten Elektroden müssen eine Vielzahl von Biegebewegungen aushalten, die durch körpereigene Veränderungen wie beispielsweise den Herzschlag hervorgerufen werden. Die Piezokristalle und Formgedächtnismetalle neigen dazu sich nach einer gewissen Anzahl von passiven Biegebewegungen durch die Elektrodenhülle der Elektrodenleitung hindurchzuarbeiten.

Es ist daher Aufgabe der vorliegenden Erfindung, eine Elektrodenleitung der eingangs genannten Art bereitzustellen, die dauerhaft implantierbar ist und einen geeigneten Steuermechanismus aufweist.

Die Erfindung wird in Anspruch 1 definiert. Alle Ausführungsbeispiele, die nicht im Einklang mit dem Gegenstand des Anspruchs 1 sind, sind nicht Bestandteil der Erfindung.

Weiterhin wird eine Elektrodenleitung bevorzugt, die im Bereich der elektrostriktiven Strukturelemente einen Aufbau und/oder eine Formgebung besitzt, die eine Auslenkung (Biegung) der Elektrodenleitung in diesem Bereich erleichtert. Ein entsprechend vorteilhafter Aufbau der Elektrodenleitung kann beispielsweise eine helixförmige elektrische Zuleitung für die (Stimulations- oder Defibrillations-) Elektrode umfassen, deren Windungen im Bereich der elektrostriktiven Strukturelemente voneinander beabstandet sind, um die Flexibilität der Elektrodenleitung zu erhöhen. Alternativ oder zusätzlich kann die Elektrodenleitung im Bereich des eines Innenradiuses der vorgesehenen Biegung oder alternativ oder zusätzlich im Bereich eines Außenradiuses der durch die elektrostriktiven Strukturelemente zu bewirkenden Biegung die Biegsamkeit erhöhende Einkerbungen besitzen.

Die elektrische Zuleitung zum Verbinden des elektrostriktiven Polymers mit der Spannungsquelle erstreckt sich vorzugsweise vom proximalen Ende der Elektrodenleitung zu dem Strukturelement. Die Spannungsquelle kann deshalb örtlich getrennt von dem distalen Ende der Elektrodenleitung platziert werden. Um die Anzahl elektrischer Zuleitungen zu begrenzen ist es möglich, eine elektrische für die (Stimulations- oder Defibrillations-) Elektrode als elektrische Zuleitung für die elektrostriktiven Strukturelemente zu verwenden. Besonders bevorzugt ist es, wenn diese Elektroden-Zuleitung ausgebildet ist, für mehrere elektrostriktive Strukturelemente ein gemeinsames Bezugspotenzial zur Verfügung zu stellen.

Die Elektrodenleitung umfasst vorzugsweise eine flexible Elektrodenhülle, die die Elektrodenleitung im Bereich des distalen Endes ummantelt, wobei das Strukturelement in die Elektrodenhülle integriert ist. Das elektrostriktive Polymer des Strukturelements ist ein flexibler Stoff, der problemlos als Bestandteil der Elektrodenhülle ausgebildet werden kann. Bevorzugt wird ein elektrostriktives Material mit sehr geringer Leitfähigkeit verwendet, um einen Stromfluss in umliegendes Gewebe zu verhindern. Das Gleiche gilt für die eingesetzte flexible Elektrodenhülle. Die Integration des Strukturelements in die Elektrodenhülle lässt eine Miniaturisierung der Elektrodenleitung zu. Dies ist insbesondere dann erforderlich, wenn die Elektrodenleitung in kleine Hohlräume, beispielsweise Gefäße mit geringem Durchmesser geführt werden soll.

Vorzugsweise ist das Strukturelement im Bereich des distalen Endes der Elektrodenleitung und neben deren Längsachse untergebracht. Die Anordnung des Strukturelements neben der Längsachse hat den Vorteil, dass eine Krümmung des distalen Endes gegenüber der Längsachse selbst bei kleinen Verformungen des Strukturelements bereits sehr groß ist. Ausführungsbeispiele der vorliegenden Erfindung werden nachfolgend anhand der beigefügten Zeichnungen beschrieben.

Es kann auch vorteilhaft sein, eine Gruppe der Strukturelemente gruppenweise ansteuerbar auszubilden. Die Strukturelemente einer der Gruppen werden dazu beispielsweise über eine gemeinsame elektrische Zuleitung mit der Spannungsquelle verbunden. Damit wird die Anzahl der elektrischen Zuleitungen gleichzeitig reduziert. Wird von der Spannungsquelle ein Signal auf die gemeinsame elektrische Zuleitung gelegt, so gelangt dieses Signal zu jeder der elektrischen Zuleitungen und bewirkt eine gleichzeitige Verformung der Strukturelemente. Eine Gruppe der Strukurelemente kann derart ausgewählt sein, dass sie lediglich Strukturelemente umfasst, die in Richtung der Längsachse voneinander beabstandet sind. Durch das gleichzeitige Ansteuern wird die Elektrodenleitung an unterschiedlichen Stellen ihrer Längsachse gleichzeitig verbogen. Eine weitere mögliche Auswahl von Strukturelementen einer Gruppe kann diejenigen Strukturelemente umfassen, die in unterschiedlichen Sektoren des Querschnitts der Elektrodenleitung angeordnet sind. Wird diese Gruppen von Strukturelementen gleichzeitig angesteuert, so wird die Elektrodenleitung in unterschiedlichen Sektoren in Umfangrichtung der Elektrodenleitung gleichzeitig verformt.

Es zeigen:
- Figur 1: eine Elektrodenleitung im Bereich des distalen Endes
- Figur 2: eine Elektrodenleitung im Bereich des distalen Endes gemäß dem zweiten Ausführungsbeispiel der vorliegenden Erfindung.

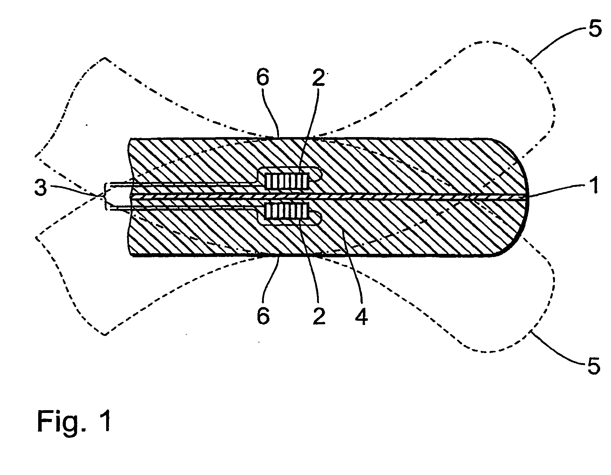

Die in Figur 1 dargestellte Elektrodenleitung ist nicht Bestandteil der Erfindung.

Figur 1 zeigt eine seitliche Schnittansicht einer Elektrodenleitung. Die Elektrode 1 erstreckt sich entlang der Längsachse der Elektrodenleitung und ist von einer flexiblen Elektrodenhülle 4 umgeben. Die gestrichelten Linien zeigen den Umriss der Elektrodenleitung, wenn diese gekrümmt worden ist. Dazu später mehr. Zwei Strukturelemente 2 sind auf entgegengesetzten Seiten von der Elektrode 1 in der Elektrodenhülle 4 integriert. Die Strukturelemente 2 sind jeweils an zwei elektrische Zuleitungen 3 angeschlossen, die zu dem (nicht gezeigten) proximalen Ende der Elektrodenleitung führen und dort an eine Spannungsquelle angeschlossen sind. Beide Strukturelemente 2 können getrennt angesteuert werden. Die Strukturelemente 2 weisen jeweils ein elektrostriktives Polymer auf, welches seine Form bei Anlegen einer Spannung verändert. Im vorliegenden Ausführungsbeispiel bewirkt das Anlegen einer Spannung an eine der beiden Strukturelemente 2, dass diese entlang der Richtung der Längsachse kontrahiert werden. Aufgrund der zusammenhängenden Einbettung der Strukturelemente 2 in die elastische Elektrodenhülle 4 bewirkt die Kontraktion im Bereich des Strukturelements 2, dass die Elektrodenleitung insgesamt verformt wird. Das Ende der Elektrodenleitung wird gegenüber der Längsachse zu dem Strukturelement 2 hin gekrümmt. Die Krümmung 6 im Bereich der Strukturelemente 2 ist maximal. Vorteilhaft an dieser Auführungsform ist, dass das distale Ende der Elektrode 2 in einander entgegengesetzte Richtungen gekrümmt werden kann.

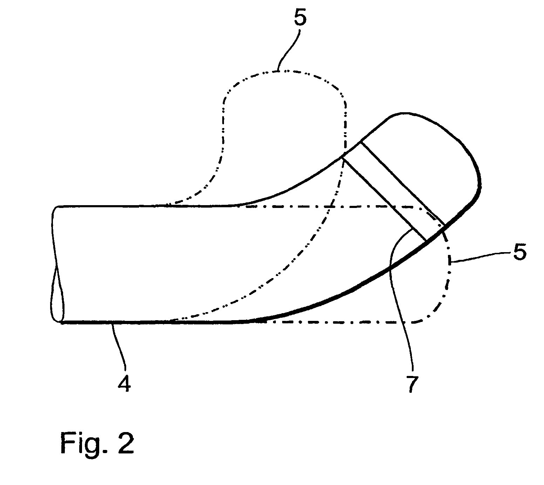

Figur 2 zeigt ebenfalls ein distales Ende einer Elektrodenleitung. Die Anordnung der Elektrode 1, der Strukturelemente 2 und der elektrischen Zuleitungen 3 im Inneren der Elektrodenleitung ist identisch mit der in Figur 1 dargestellten Anordnung und deshalb nicht dargestellt. Bezugszeichen 4 zeigt wiederum die flexible Elektrodenhülle, wobei keine Spannung an den Strukturelementen anliegt. Die Elektrodenhülle 4 bzw. das distale Ende der Elektrode ist im Gegensatz zu der in Figur 1 gezeigten Elektrodenleitung in diesem Zustand gekrümmt. Bezugszeichen 5 zeigt wiederum den Umriss der Elektrodenleitung, der entstehen kann, wenn an die Strukturelemente 3 eine Spannung angelegt wird. Das distale Ende wird von den Strukturelementen 2 soweit verformt, dass es keine Krümmung gegenüber der Längsachse der Elektrode aufweist. Die Krümmung kann ferner derart verstärkt werden, dass das distale Ende gegenüber der Längsachse der Elektrodenleitung um einen Winkel von 90° geneigt ist.

In Figur 2 ist außerdem eine Ringelelektrode 7 dargestellt, die üblicherweise über eine elektrische Leitung mit einem Herzschrittmacher oder einem anderen Therapiegerät verbunden werden kann und beispielsweise dem Stimulieren oder dem Abfühlen elektrischer Signale des Herzgewebes dient.

## Patentansprüche

1. Elektrodenleitung, insbesondere für eine implantierbare Elektrostimulationsvorrichtung, die zum Einführen in Körpergefäße wie Herzkammem oder Blutgefäße ausgebildet ist, mit wenigstens
einer Elektrode (7) zum Stimulieren oder Abfühlen von Körpergewebe,
einer Längsachse und einem distalen Ende,
einer Veizahl von ein elektrostriktives Polymer aufweisenden Strukturelementen (2) im Bereich des distalen Endes, welche unabhängig voneinander über zumindest eine elektrische Zuleitung (3) mit einer Spannungsquelle verbindbar und von der Spannungsquelle ansteuerbar sind und ausgebildet sind, bei Anliegen einer elektrischen Spannung ihre Form zu verändem,
**dadurch gekennzeichnet,**
**dass** die Elektrodenleitung zumindest im Bereich der Strukturelemente (2) vorgekrümmt ausgebildet ist, falls keine Spannung an den Steuerelementen (2) anliegt, und die Strukturelemente (2) so ausgebildet sind, dass ein Anlegen einer Spannung an eines der Strukturelemente (2) eine Streckung der Elektrodenleitung bewirkt, während ein Anlegen einer Spannung an ein anderes Strukturelement (2) eine stärkere Krümmung (6) der Elektrodenleitung bewirkt.

2. Elektrodenleitung nach Anspruch 1, **dadurch gekennzeichnet, dass** das Strukturelement (2) ausgebildet und bezüglich der übrigen Elektroden (7) derart angeordnet ist, dass eine Krümmung (6) der Längsachse der Elektrodenleitung im Bereich des distalen Endes durch Anlegen einer elektrischen Spannung veränderbar ist.

3. Elektrodenleitung nach Anspruch 1 oder 2, **dadurch gekennzeichnet, dass** zwei der Strukturelemente (2) bezüglich eines Querschnitts der Elektrodenleitung auf gegenüberliegenden Seiten des Querschnittes angeordnet sind, derart das wahlweises Anlegen elektrischer Spannung an eines der Strukturelemente (2) eine Krümmung der Elektrodenleitung in eine andere Richtung bewirkt, als ein Anlegen der Spannung an das jeweils andere Strukturelement.

4. Elektrodenleitung nach Anspruch 1, 2 oder 3, **dadurch gekennzeichnet, dass** mehrere der Strukturelemente (2) jeweils bezüglich des Querschnitts der Elektrodenleitung einen Sektor in Umfangsrichtung der Elektrodenleitung einnehmen.

5. Elektrodenleitung nach einem der Ansprüche 1 bis 4, **dadurch gekennzeichnet, dass** sich die elektrische Zuleitung (3) vom proximalen Ende der Elektrodenleitung zu dem Strukturelement (2) erstreckt.

6. Elektrodenleitung nach einem der vorstehenden Ansprüche, **gekennzeichnet durch** eine flexible Elektrodenhülle (4), die die Elektroden ummantelt, wobei das Strukturelement (2) in die Elektrodenhülle (4) integriert ist.

7. Elektrodenleitung nach einem der vorstehenden Ansprüche, **dadurch gekennzeichnet, dass** das Strukturelement (2) im Bereich des distalen Endes neben der Längsachse angeordnet ist.

8. Elektrodenleitung nach Anspruch 1, 2, 3 oder 4, **dadurch gekennzeichnet, dass** die Vielzahl der Strukturelemente (2) derart mit der Spannungsquelle verbindbar sind, dass die Vielzahl der Strukturelemente (2) in Gruppen von der Spannungsquelle ansteuerbar sind.

9. Elektrodenleitung nach Anspruch 8, **dadurch gekennzeichnet, dass** eine elektrische Zuleitung für die Elektrode (7) als elektrische Zuleitung eines oder mehrere Strukturelemente ausgebildet ist.

10. Elektrodenleitung nach Anspruch 8 oder 9, **dadurch gekennzeichnet, dass** eine erste Gruppe der Strukturelemente (2) entlang der Längsachse voneinander beabstandete Strukturelemente (2) umfasst.

11. Elektrodenleitung nach einem der Ansprüche 8 bis 10, **dadurch gekennzeichnet, dass** eine zweite Gruppe der Strukturelemente (2) in unterschiedlichen Radialsektoren angeordnete Strukturelemente (2) umfasst.

12. Elektrodenleitung nach Anspruch 8, 9, 10 oder 11, **dadurch gekennzeichnet, dass** ausgewählte Strukturelemente (2) einer der Gruppen **dadurch** gemeinsam ansteuerbar sind, dass sie über eine gemeinsame elektrische Leitung mit der Spannungsquelle verbindbar sind.

13. Elektrodenleitung nach Anspruch 1, **gekennzeichnet durch** eine die Biegsamkeit der Elektrodenleitung im Bereich der elektrostriktiven Strukturelemente erhöhende Formgebung und/oder einen diese Biegsamkeit erhöhenden Aufbau im Bereich der elektrostriktiven Strukturlemente.

## Claims

1. Electrode line, in particular for an implantable electrostimulation device, which is configured for insertion into body vessels, such as ventricles or blood vessels, comprising at least one electrode (7) for stimulating or sensing body tissue, a longitudinal axis and a distal end, a large number of structural elements (2) comprising an electrostrictive polymer in the region of the distal end, which structural elements can independently of one another be connected to a voltage source via at least one electrical feed line (3), which can be actuated by the voltage source and which are configured to change their shape when an electrical voltage is applied, **characterised in that** the electrode line is of a curved configuration, at least in the region of the structural elements (2), when no voltage is applied to the structural elements (2), and the structural elements are configured such that application of a voltage to one of the structural elements (2) causes straightening of the electrode line, while application of a voltage to another structural element (2) causes a greater curvature (6) of the electrode line.

2. Electrode line according to claim 1, **characterised in that** the structural element (2) is configured and is arranged with respect to the rest of the electrodes (7) such that a curvature (6) of the longitudinal axis of the electrode line, in the region of the distal end, can be varied by applying an electrical voltage.

3. Electrode line according to either claim 1 or claim 2, **characterised in that** two of the structural elements (2) are arranged on opposing sides of a cross-section of the electrode line, such that the selective application of electrical voltage to one of the structural elements (2) causes curvature of the electrode line in a direction different from application of the voltage to the respective other structural element.

4. Electrode line according to claim 1, 2 or 3, **characterised in that** each of a plurality of the structural elements (2) occupies a respective sector in the peripheral direction of the electrode line with respect to the cross-section thereof.

5. Electrode line according to any one of claims 1 to 4, **characterised in that** the electrical feed line (3) extends from the proximal end of the electrode line to the structural element (2).

6. Electrode line according to any one of the preceding claims, **characterised by** a flexible electrode sheath (4) encasing the electrodes, wherein the structural element (2) is integrated into the electrode sheath (4).

7. Electrode line according to any one of the preceding claims, **characterised in that** the structural element (2) is arranged next to the longitudinal axis in the region of the distal end.

8. Electrode line according to claim 1, 2, 3 or 4, **characterised in that** the large number of structural elements (2) can be connected to the voltage source so as to be actuable in groups by the voltage source.

9. Electrode line according to claim 8, **characterised in that** an electrical feed line for the electrode (7) is configured as the electrical feed line of one or more structural elements.

10. Electrode line according to claim 8 or claim 9, **characterised in that** a first group of the structural elements (2) comprises structural elements (2) which are spaced from one another along the longitudinal axis.

11. Electrode line according to any one of claims 8 to 10, **characterised in that** a second group of the structural elements (2) comprises structural elements (2) arranged in different radial sectors.

12. Electrode line according to claim 8, 9, 10 or 11, **characterised in that** selected structural elements (2) of one of the groups are co-actuable by virtue of being connectable to the voltage source via a common electrical line.

13. Electrode line according to claim 1, **characterised by** a configuration which increases the flexibility of the electrode line in the region of the electrostrictive structural elements and/or a structure which increases said flexibility in the region of the electrostrictive structural elements.

## Revendications

1. Fil d'électrode, en particulier pour un électrostimulateur intégré, lequel est conçu pour être introduit dans des cavités naturelles, telles que les ventricules ou les vaisseaux sanguins, comportant au moins
- une électrode (7) destinée à stimuler ou détecter les tissus physiologiques,
- un axe longitudinal et une extrémité distale,
- une pluralité d'éléments structurels (2) en polymère électrostrictif, qui sont disposés dans la zone de l'extrémité distale et qui, indépendamment les uns des autres, peuvent être reliés à une source de tension via au moins un câble d'alimentation électrique (3) et peuvent être commandés par la source de tension et sont conçus pour modifier leur forme sous l'effet de l'application d'une tension électrique,
**caractérisé en ce que**
le fil d'électrode est pré-cintré au moins dans la zone des éléments structurels (2) si aucune tension n'est appliquée sur les éléments structurels (2), et les éléments structurels (2) sont conçus de telle sorte que l'application d'une tension sur un des éléments structurels (2) entraîne une extension du fil d'électrode, alors que l'application d'une tension sur un autre élément structurel (2) provoque un cintrage (6) plus accentué du fil d'électrode.

2. Fil d'électrode selon la revendication 1, **caractérisé en ce que** l'élément structurel (2) est conçu et agencé par rapport aux autres électrodes (7) de telle sorte qu'un cintrage (6) de l'axe longitudinal du fil d'électrode dans la zone de l'extrémité distale est modifiable par l'application d'une tension électrique.

3. Fil d'électrode selon la revendication 1 ou 2, **caractérisé en ce que** deux des éléments structurels (2) sont agencés, par rapport à une coupe transversale du fil d'électrode, sur des côtés face à face de la coupe transversale, de telle sorte que l'application optionnelle d'une tension électrique sur un des éléments structurels (2) engendre un cintrage du fil d'électrode dans une direction différente de la direction de cintrage lors de l'application d'une tension électrique sur respectivement l'autre élément structurel.

4. Fil d'électrode selon la revendication 1, 2 ou 3, **caractérisé en ce que** plusieurs des éléments structurels (2) couvrent respectivement, par rapport à la coupe transversale du fil d'électrode, un secteur dans le sens périphérique du fil d'électrode.

5. Fil d'électrode selon l'une quelconque des revendications 1 à 4, **caractérisé en ce que** le câble d'alimentation électrique (3) s'étend de l'extrémité proximale du fil d'électrode vers l'élément structurel (2).

6. Fil d'électrode selon l'une quelconque des revendications précédentes, **caractérisé par** une enveloppe d'électrode (4) flexible qui entoure les électrodes, l'élément structurel (2) étant intégré dans l'enveloppe d'électrode (4).

7. Fil d'électrode selon l'une quelconque des revendications précédentes, **caractérisé en ce que** l'élément structurel (2) est agencé dans la zone de l'extrémité distale à côté de l'axe longitudinal.

8. Fil d'électrode selon la revendication 1, 2, 3 ou 4, **caractérisé en ce que** la pluralité d'éléments structurels (2) sont reliés à la source de tension, de telle sorte que la pluralité d'éléments structurels (2) peuvent être commandée par groupes par la source de tension.

9. Fil d'électrode selon la revendication 8, **caractérisé en ce qu'**un câble d'alimentation électrique pour l'électrode (7) est réalisé sous forme de câble d'alimentation électrique d'un ou de plusieurs éléments structurels.

10. Fil d'électrode selon la revendication 8 ou 9, **caractérisé en ce qu'**un premier groupe d'éléments structurels (2) comporte des éléments structurels (2) écartés les uns des autres le long de l'axe longitudinal.

11. Fil d'électrode selon l'une quelconque des revendications 8 à 10, **caractérisé en ce qu'**un deuxième groupe d'éléments structurels (2) comporte des éléments structurels (2) agencés dans différents secteurs radiaux.

12. Fil d'électrode selon la revendication 8, 9, 10 ou 11, **caractérisé en ce que** des éléments structurels (2) sélectionnés de l'un des groupes peuvent être commandés conjointement par le fait qu'ils peuvent être reliés à la source de tension via une ligne électrique commune.

13. Fil d'électrode selon la revendication 1, **caractérisé par** un formage augmentant la flexibilité du fil d'électrode dans la zone des éléments structurels électrostrictifs et/ou par une structure augmentant cette flexibilité dans la zone des éléments structurels électrostrictifs.
